# EUROPEAN PATENT APPLICATION

(11) **EP 0 774 231 A1**
(43) Date of publication of application: **21.05.1997**
(21) Application number: 96307070.1
(22) Date of filing: 27.09.1996
(51) Int. Cl.: A61B 1/00

(54) **Battery powered electronic video endoscope**

(30) Priority: 20.11.1995 US 560903
(71) Applicant: WILSON GREATBATCH LTD., Clarence New York 14031 (US)
(72) Inventor: Mott, Richard W., East Armherst, NY 14051 (US); Siegler, Robert W., Williamsville, NY 14221 (US)
(74) Representative: Smaggasgale, Gillian Helen

(57) **Abstract**

A heat sterilizable electronic video endoscope powered by a high energy/power density battery, is described. Optical images of an investigation site are converted to electrical signals which are transmitted by radio transceivers from the endoscope to a remote video processor. The video processor converts the radio signals to a video image viewable on a TV monitor. All of the electrical components of the endoscope are powered by the battery which is detachable therefrom for replacement. The battery and the optical and electrical components of the endoscope are selected to withstand the temperatures of steam autoclave sterilization.

## Description

### BACKGROUND OF THE INVENTION

### 1. Field Of The Invention

The present invention generally relates to a sterilizable electronic video endoscope, and more particularly to a heat sterilizable electronic video endoscope. A preferred embodiment of the present video endoscope is powered by a high energy/power density battery.

### 2. Prior Art

Video endoscopes have been used in surgical procedures for several decades. Typically, video endoscopes include a tubular housing supporting an objective lens system at a distal end thereof. A charge coupled device (CCD) is mounted in the tubular housing in optical relation to the objective lens system for sensing a light image and converting it to an electrical signal of the image which is processed by circuitry and transmitted by an electrical cable to a video monitor. Optical fibers coupled to a light source located remotely from the video endoscope extend to the distal end of the tubular housing to emit an illuminating light on an object under investigation and intended to be imaged.

Because of their many optical and electronic components in addition to their light transmitting glass fibers, endoscopes are very expensive instruments that normally are not disposable. Therefore, in order to reuse the endoscope, it needs to be sterilized between uses to minimize the possibility of the endoscope serving as a source of infection and as a carrier of disease. One method is to soak the endoscope for several hours in a cold sterilant such as peracetic acid. However, this practice is presently being scrutinized in its ability to actually sterilize. An alternative, when possible, is to heat sterilize the endoscope in a steam autoclave.

An electronic video endoscope intended to be heat sterilized between uses is described in U.S. Patent No. 5,188,094 to Adair. This instrument includes heat shielding glass fibers for illumination of the target object. The glass fibers are packed densely circumferentially around the centrally located and heat resistant lens system and electronic circuitry to further protect them from the heat of sterilization. The electronic circuitry is connectable to a VCR and a TV monitor by an electrical cable for recording and viewing the site under investigation.

U.S. Patent No. 5,381,784 to Adair describes a stereoscopic endoscope that, in part, provides a sterilizable instrument by densely packing the glass illumination fibers circumferentially around the centrally located and heat resistant lens system and electronic circuitry. As is the case with the endoscope described in the '094 Adair patent, the glass fibers help shield the internal components from.the heat of sterilization. Again, an electrical cable having image signal transmitting wires connects between the electronic circuitry and a remote electronic control unit associated with a TV monitor for viewing the site of interest.

While these prior art devices perform satisfactorily for their intended purpose, there is a need for an endoscope that is both readily heat sterilizable between uses and that does not require or include a transmission cable connecting the instrument to a video processor for the VCR and TV monitor. This latter feature would eliminate the tethering effect of the transmission cable and, in part, allow the physician or attendant to more freely move about the operating theater.

### SUMMARY OF THE INVENTION

The present invention relates to an endoscope provided with a battery mounted on the endoscope as its sole means of electrical power.

In a second aspect of the present invention there is provided a heat sterilizable video endoscope which includes a tubular housing having a distal end intended to be inserted into a body cavity and a proximal end. An objective lens system comprising a plurality of focused lens and a CCD for electronically sensing the image of the investigation site within the body cavity are preferably located inside the distal end of the tubular housing. Light transmitting optical fibers coupled to a remote white light source preferably extend through the tubular housing to the distal end thereof for illuminating the investigation site. Electrical wire leads connected to the CCD may transmit electrical image signals to radio frequency transceiver components located in the proximal end of the tubular housing. The electrical image signals are preferably processed by a remote radio video processor having a monitor which enables the physician to view a representation of the investigation site. The transceivers preferably also receive telemetry control signals from the video processor. In that manner, the video processor regulates the telemetry of the received image signals and the transmitted control signals to provide a clear and well defined image on the monitor. Power is preferably provided to the electrical components of the endoscope including the transceiver components and the CCD by an autoclavable high energy/power density battery which may be detachably mounted on the distal end of the tubular housing. Various embodiments of battery powered stereoscopic endoscopes capable of providing the physician with a three dimensional view of the investigation site are also disclosed.

The present video endoscopes are an improvement over currently available endoscopes in that the provision of a battery to power the electrical components eliminates the electrical cable connected to the video processor for the VCR and the TV monitor. This helps to free the movement of the physician in the operating theater unhindered by the electrical cable that in the prior art devices transmits the image signal between the electronic circuitry of the endoscope and the remote electronic control unit associated with the TV monitor. The autoclavable high energy/power density battery along with the autoclavable nature of the other components enable the various endoscopes of the present invention to be capable of multiple uses before replacement is required.

The above aspects of the present invention will become more apparent to those skilled in the art by reference to the following description and to the following drawings in which particular embodiments of the invention are described.

### BRIEF DESCRIPTION OF THE DRAWINGS

Fig. 1 is a schematic view of a battery powered video endoscope 10 of the present invention and an associated remote video processor 48 connected to a TV monitor 50.

Fig. 2 is a cross-sectional view with parts broken away of the endoscope 10 including a detachably mounted high energy/power density battery 24 according to the present invention.

Fig. 3 is a cross-sectional view with parts broken away of the distal end of one embodiment of a stereoscopic endoscope according to the present invention.

Fig. 4 is a cross-sectional view with parts broken away of the distal end of another embodiment of a stereoscopic endoscope according to the present invention.

Fig. 5 is a cross-sectional view with parts broken away of the distal end of still another embodiment of a stereoscopic endoscope according to the present invention.

Fig. 6 is a cross-sectional view of the distal end of a further embodiment of a stereoscopic endoscope having a hinged end cap in a closed position.

Fig. 7 is an end view of the stereoscopic endoscopic shown in Fig. 6 with the hinged end cap in an opened position.

### DETAILED DESCRIPTION OF PREFERRED EMBODIMENTS OF THE INVENTION

Throughout the figures showing the various embodiments of the battery powered, sterilizable video endoscopes of the present invention, like members are designated with like numerals.

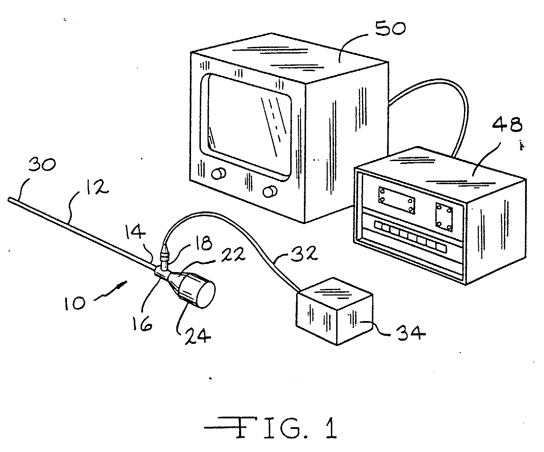

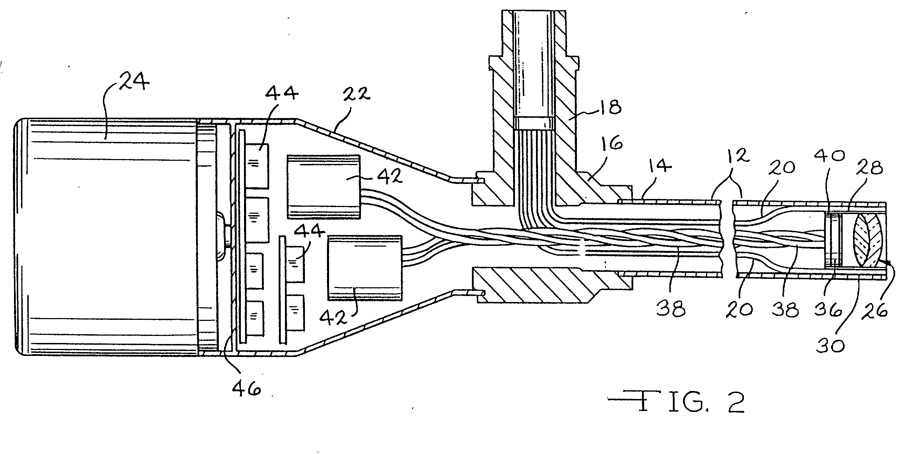

Referring now to the drawings, Figs. 1 and 2 show a battery powered, sterilizable video endoscope 10 according to one embodiment of the present invention. The endoscope 10 comprises a conduit housing 12 having a tubular shape. The tubular housing 12 is preferably made of metal and it can be rigid, as shown, or the housing can be flexible and constructed by pivotally connecting the mutually facing ends of a plurality of juxtaposed rings, as is well known to those skilled in the art. A proximal end 14 of the housing 12 is joined to a T-shaped connector 16 having a light guide post 18 through which optical glass fibers 20 are disposed. An adaptor 22 is joined to the connector 16 opposite the tubular housing 12. The adaptor 22 serves to detachably mount a battery 24 for supplying electrical power to the various electronic components of the endoscope 10, as will be explained in detail hereinafter. Adaptor 22 is an extensidn of the proximal end 14 of housing 12.

Objective optics comprising a lens system 26 are housed in a cylindrically shaped optics fitting 28 disposed inside the distal end 30 of the tubular housing 12. The optical glass fibers 20 begin in the T-shaped connector 16 at the guide post 18 and extend the length of the tubular housing 12 to the distal end 30 thereof. There, they are positioned in a tightly packed manner circumferentially around the optics fitting 28 to confine the optics fitting 28 centered inside the tubular housing 12.

In use, a fiber optic light cable 32 attaches to guide post 18 and supplies light from a remote white light source 34 to the optical glass fibers 20 which in turn transmits the light to the distal end 30 of the tubular housing 12 from which the light is directed onto the site under investigation. A visual image is then reflected from the investigation site through the lens system 26 and to an associated charge coupled device 36, also known as a CCD, for sensing the image. The CCD 36 converts the visual image into electrical signals that are transmitted through electrical leads 38 via an electronic package 40 to a pair of radio frequency transceiver components 42 located in the adaptor 22.

The light source 34 is a relatively compact component that is positionable closely adjacent to the investigation site. In that manner, the light cable 32 does not act as a hinderance to movement of the physician about the operating theater.

Electrical power is supplied from the battery 24 to the endoscope 10 including the transceivers 42 through electronic interface circuits on boards 44, some of which are mounted on an end wall 46 of the adaptor 22. The electrical signals containing information on images of the investigation site are transmitted by the transceivers 42 as electromagnetic radio waves to a remote video processor 48. There, the radio waves are converted to a video image viewable on a TV monitor 50 connected to the processor 48. Although not shown, the monitor 50 may include a VCR for recording the video image. The video processor 48 also transmits electromagnetic radio waves back to the transceivers 42 as telemetry control signals for controlling the operation thereof in a known manner.

The battery 24 is preferably detachable from the endoscope 10 for replacement at such time as its energy capacity has been depleted to an extent where it is no longer safe for use during the investigation or operative procedure. In accordance with the present invention, the battery 24 is sterilizable in a steam autoclave (not shown) along with the endoscope 10. For that reason, both the endoscope 10 and battery 24 are constructed to remain hermetically sealed over a broad range from about -70 cm Hg to about 2 to 3 atmospheres, and at autoclave temperatures as high as about 135°C. A suitable autoclavable battery is manufactured by Wilson Greatbatch Ltd., Clarence, New York under Model No. HTS C/D. By way of example, in an illustrative endoscope wherein the components are selected to be able to withstand the temperatures of autoclave sterilization, optical fibers 20 are of the type commercially available from Fiber Optic Technologies located in Connectic.ut under the designation White Light Fiber, lens system 26 and optics fitting 28 are custom configured and commercially available from P.O.C. located in Massachusetts, charge coupled device 36 is commercially available from Panasonic under the designation KS 152, and the transceivers 40 are commercially available from Motorola. The circuit components on boards 44 are either selected to withstand the autoclave sterilizations or are encapsulated within a potting compound which can withstand such temperatures.

In accordance with further aspects of the present invention, several embodiments of stereoscopic endoscopes are shown in Figs. 3 to 7.

Referring to Fig. 3, there is shown a first embodiment of a battery powered, sterilizable stereoscopic endoscope 60 having a tubular conduit housing 62 preferably made of metal. As is the case with the endoscope 10 shown in Figs. 1 and 2, the housing 62 for the stereoscopic endoscope 60 can be rigid, as shown, or the housing can be flexible. Objective optics comprising a lens system 64 are housed in a cylindrically shaped optics fitting 66 disposed inside the distal end 68 of the tubular housing 62. Optical glass fibers 70 are positioned in a tightly packed manner circumferentially around the optics fitting 66 to confine the optics fitting centered inside the tubular housing. While not shown in Fig. 3, the proximal end of the optical glass fibers 70 extend the length of the tubular housing 62 to a guide post attachable to a fiber optic light cable that supplies light from the remote white light source 34 (Fig. 1), in a similar manner as previously described with respect to the endoscope 10.

The light is directed from the optical glass fibers 70 onto the site under investigation and a visual image is then reflected from the investigation site through the lens system 64 and to a pair of side-by-side CCDs 72, 74. The CCDs 72, 74 are separated and retained in place by a metal spacer 76 disposed therebetween. The CCDs convert the visual image into electrical signals that are transmitted through electrical leads 78 via respective electronic packages 80, 82 to the previously described radio frequency transceiver components 42 (Fig. 2). The pair of CCDs 72, 74 serve to divide the reflected image, and the electronic packages 80, 82 and transceivers in turn transmit electromagnetic radio waves to the remote video processor 48 related to the two portions of the divided reflected image. The video processor 48 also transmits electromagnetic radio waves back to the transceivers as telemetry control signals for controlling-the operation thereof in a known manner. That way, the stereoscopic endoscope 60 provides the physician with a clear and well defined three-dimensional view of the investigation site on the TV monitor 50.

As with the previously described endoscope 10, electrical power is supplied from a battery, such as battery 24 (Figs. 1 and 2), to power the endoscope 60 including transceivers 42. Again, the battery for the stereoscopic endoscope 60 is preferably detachable therefrom for replacement and so that it can be sterilized separate from the endoscope 60. The components comprising the stereoscopic endoscope 60 are selected to be sterilizable, preferably by steam or heat sterilization.

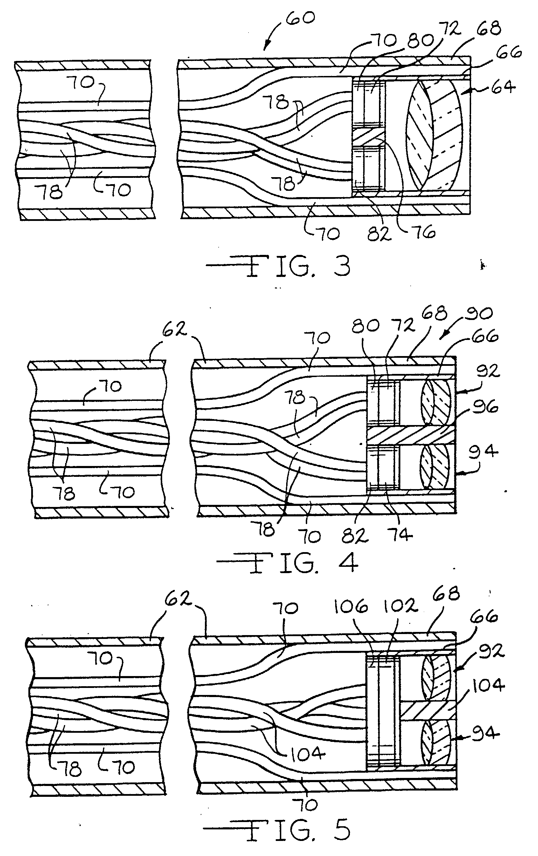

Fig. 4 shows the distal end of another embodiment of a battery powered, sterilizable stereoscopic endoscope 90 according to the present invention. This stereoscopic endoscope differs from the stereoscopic endoscope 60 shown in Fig. 3 in that the visual image reflected from the investigation site is returned to objective optics comprising a pair of side-by-side lens systems 92, 94. The reflected light passes through the side-by-side lens systems 92, 94 to the pair of dedicated side-by-side CCDs 72, 74 that convert the respective received visual images into electrical signals that are transmitted through electrical leads 78 via the associated electronic packages 80, 82 to radio frequency transceivers 42 (Fig. 2). The lens systems 92, 94 serve to divide the reflected image into two portions which the CCDs 72, 72 and the electronic packages 80, 82 in conjunction with the transceivers transmit as electromagnetic radio waves to the remote video processor 48 to provide the physician with a three-dimensional view of the investigation site on the TV monitor 50. As is the case with the previously described endoscopes 10, 60, the video processor 48 also transmits electromagnetic radio waves to the transceivers for telemetry control. The lens systems 92, 94, the CCDs 72, 74 and the associated electronic packages 80, 82 are separated and retained in place by a metal spacer 96 disposed therebetween.

A detachable battery (not shown) is provided for powering the stereoscopic endoscope 90. Preferably, the battery and the various components comprising the endoscope 90 are selected to be sterilizable such as in a steam autoclave.

Fig. 5 shows the distal end of still another embodiment of a battery powered, sterilizable stereoscopic endoscope 100 according to the present invention. In this stereoscopic endoscope 100, the visual image reflected from the investigation site is returned and reflected through objective optics comprising the pair of side-by-side lens systems 92, 94 and a single dedicated CCD 102. The lens systems 92, 94 are separated and retained in place by a metal spacer 104 disposed therebetween. In this embodiment of the present stereoscopic endoscope, the CCD 102 is configured to convert the visual image from the lens system pair 92, 94 into electrical signals that are transmitted as discrete data packets related to that portion of the reflected image captured by each lens system 92, 94 through electrical leads 104 via an electronics package 106 to the radio frequency transceiver components 42 (Fig. 2). The transceivers 42 then transmit electromagnetic radio waves of the visual images from the lens systems 92, 94 to the remote video processor 48 to permit the physician to view a three-dimensional representation of the investigation site on the TV monitor 50. Control telemetry is also transmitted from the processor 48 to the transceivers 42. Again, a detachable and autoclavable sterilizable battery (not shown) is provided to power the stereoscopic endoscope 100 whose components are selected to be heat or steam sterilizable.

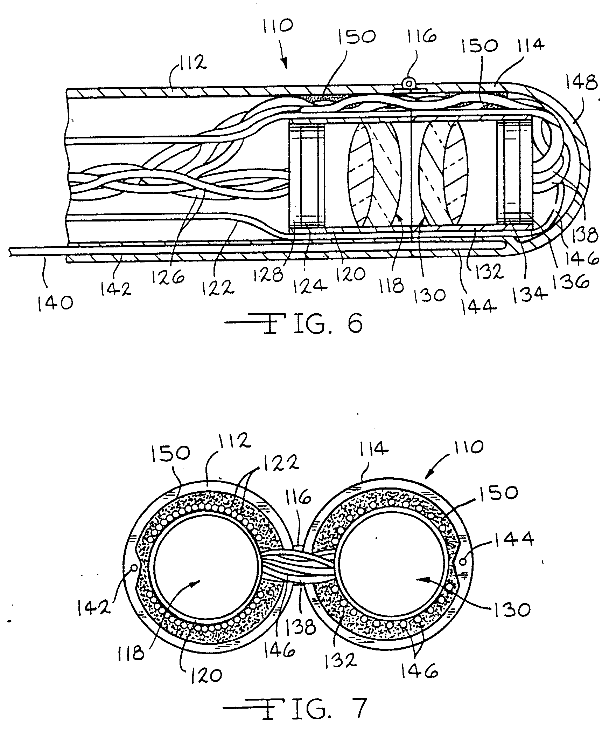

Figs. 6 and 7 show a further embodiment of the distal end of a battery powered, sterilizable stereoscopic endoscope 110 according to the present invention. Stereoscopic endoscopic 110 includes a tubular conduit housing 112, preferably made of metal, having an end cap 114 pivotally attached by a hinge 116 to the distal end thereof. The hinge 116 is normally biased into an opened position by means of a coil spring (not shown) extending around hinge 116. A first objective optics comprising a lens system 118 is positioned inside a cylindrically shaped optics fitting 120 disposed inside the distal end of the tubular housing. Optical glass fibers 122 are positioned circumferentially around the optics fitting 120 to help confine the optics fitting coaxially positioned inside the tubular housing 112. A first CCD 124 is mounted inside the fitting 120 just behind the lens system 118 to receive the light passing through the lens system 118 and to convert this light into electrical signals that are transmitted through electrical leads 126 via an electronic package 128 to the previously described radio frequency transceivers 42 (Fig. 2).

A second objective optics comprising a second lens system 130 is positioned inside a cylindrically shaped optics fitting 132 disposed inside the end cap 114. The second lens system 130 has an associated CCD 134 positioned inside the fitting 132 to receive the light passing therethrough and to convert it into electrical signals. The electrical signals from the second CCD 134 are transmitted via a second electronic package 136 by electrical leads 138 which extend through the end cap 114, past hinge 116 and along the length of the tubular housing 112 to the radio frequency transceivers 42. The transceivers 42 then transmit electromagnetic radio waves to the remote video processor 48 corresponding to the electrical signals from both the first lens system 118 located in the distal end of tubular housing 112 and the second lens system 130 located in the end cap 114 hinged to the housing 112. Control telemetry is also transmitted from the processor 48 to the transceivers for both CCDs 124, 134 associated with the first and second lens systems 118, 130.

As shown in Fig. 6, the first and second lens systems 118, 130 are in a face-to-face relationship when the end cap 114 is in a closed position on the distal end of the tubular housing 112. A locking rod 140 is mounted for sliding movement through a channel 142 extending within and along the length of the tubular housing 112. The locking rod 140 has a distal end that extends into a sleeve 144 in end cap 114 with a friction fit sufficient to retain end cap 114 in the closed position against the force of the coil spring extending around the hinge 116.

Some of the optical glass fibers 122 are positioned in a tightly packed configuration around the first optics fitting 120 for the purpose of retaining the fitting in place and for illuminating the investigation site at such time as the end cap 114 is in the opened position (Fig. 7). Additional optical glass fibers 146 extend beyond the first optics fitting 120 adjacent to the hinge 116 to the dome portion 148 of end cap 114 where they double back and fan out in a tightly packed manner along and around the second optics fitting 132 to the open end of end cap to further illuminate the investigation site with the end cap 114 in the opened position. Suitable potting material 150 is provided proximate the optical glass fibers 122, 146 adjacent to the first and second optics fittings 120, 132 to prevent circumferential movement of the optical fibers 122, 146 and the electrical leads 138 for the second electronic package 136 while providing for a sufficient amount of play in the optical fibers 146 and the leads 138 as the end cap 114 is moved between the opened and the closed positions.

Electrical power for the stereoscopic endoscope 110 is supplied from a battery, such as battery 24 (Figs. 1 and 2). As is the case with the previously described endoscopes 10, 60, 90 and 100, the battery is preferably detachable therefrom and both the battery and the endoscope are selected to be sterilizable such as in a steam autoclave.

In use, the stereoscopic endoscope 110 is inserted to the operative site and the locking rod 140 is withdrawn from the sleeve 144 so that the end cap 114 pivots about hinge 116 to the opened position shown in Fig. 7. This arrangement creates a parallax wherein lens system 118 and associated CCD 124 and electronic package 128 lie along a first axis parallel to the longitudinal axis of the tubular housing 112 and the second lens system 130 and associated CCD 134 and electronic package 136 lie along a second axis parallel to the first axis. The resulting image seen by the physician on the TV monitor 50 is a wide-angle three dimensional view of the investigation site.

When it is desired to withdraw the stereoscopic endoscope 110 from the operation site, the end cap 114 is pushed to the closed position by engagement with the end of a trocar (not shown) through which the endoscope has been introduced into the body. Alternatively, the end cap 114 is pushed to the closed position by engagement with a body passageway through which the endoscope has been introduced to the operation site.

Thus, the present invention provides all the advantages of an endoscope that is heat sterilizable so that the endoscope is reusable without serving as a source of infection and as a carrier of disease from a one patient to the next. Additionally, radio transmission of the electrical image signals of the investigation site eliminates the need for a transmission cable connected between the CCD electronics and the remote video processor. This is a significant improvement in helping free the physician for unteathered movement about the operating theater when using the present endoscope.

It is appreciated that various modifications to the inventive concepts described herein may be apparent to those skillea in the art without departing from the spirit and scope of the present invention defined by the hereinafter appended claims.

Each feature disclosed in the description, Claims and drawings may be provided independently or in any appropriate combination.

## Claims

1. An endoscope provided with a battery mounted on the endoscope as its sole means of electrical power.

2. The endoscope of Claim 1 including means for converting optical images of an investigation site into electrical signals and a transmitter means for transmitting radio signals corresponding to the electrical signals and a remote processor means for converting the radio signals into a usable form.

3. The endoscope of Claim 2 wherein the converting means comprises a charge coupled device located in the end of the endoscope disposed toward the investigation site.

4. The endoscope of Claim 2 or 3 wherein the transmitter means is a transceiver means adapted to transmit the image signals to the remote processor means and to receive control signals for the endoscope.

5. The endoscope of any one of Claims 1 to 4 wherein the battery is detachably mounted on the endoscope.

6. The endoscope of any one of Claims 1 to 5 wherein the endoscope and battery are capable of withstanding the temperature of steam autoclave sterilization.

7. A heat sterilizable electronic video endoscope, comprising:
a) a conduit housing having a distal end and a proximal end;
b) objective optics mounted within the conduit housing at the distal end thereof;
c) CCD means mounted within the conduit housing and positioned to receive an image focused on the CCD means by the objective optics;
d) transmitter means connected to the CCD means for transmitting image signals;
e) processing means for receiving the image signals and producing a visual representation of the image;
f) electrical power means mounted on the conduit housing for supplying electrical energy to the CCD and the transmitter means; and
g) light means for emitting light onto an object to produce the image.

8. The electronic video endoscope of Claim 7 wherein the objective optics and the CCD means provide a stereoscopic visual representation of the image.

9. The electronic video endoscope of Claim 7 or 8 wherein the objective optics comprises: (a) a lens system having a pair of side-by-side CCD means associated therewith to provide the stereoscope visual representation of the image; (b) a pair of side-by-side lens systems having respective side-by-side CCD means associated therewith to provide the stereoscopic visual representation of the image; or (c) a pair of side-by-side lens systems have a CCD means associated therewith to provide the stereoscopic visual representation of the image.

10. The electronic video endoscope of any one of Claims 7 to 9 wherein the signal processing means includes a VCR.

11. The electronic video endoscope of any one of Claims 7 to 10 wherein the conduit housing has a generally elongated tubular shape.

12. The electronic video endoscope of any one of Claims 7 to 11 wherein the conduit housing is rigid.

13. The electronic video endoscope of any one of Claims 7 to 11 wherein the conduit housing is flexible.

14. The electronic video endoscope of any one of Claims 7 to 13 wherein the electrical power means is detachable from the conduit housing.

15. The electronic video endoscope of any one of Claims 7 to 14 wherein the CCD means is positioned adjacent to the objective optics and is electrically connected to the transmitter means located in the proximal end of the conduit housing.

16. The electronic video endoscope of any one of Claims 7 to 15 wherein the transmitter means is a transceiver means adapted to transmit the image signals to the processing means and to receive control signals for transmission to the CCD means.

17. A heat sterilizable stereoscopic electronic video endoscope, which comprises:
a) a conduit housing having a distal end and a proximal end;
b) first objective optics mounted within the conduit housing at the distal end thereof;
c) first CCD mounted within the conduit housing and positioned to receive an image focused on it by the first objective optics;
d) an end cap hinged to the distal end of the conduit housing so that the end cap is movable between a closed position covering the distal end of the conduit housing and an opened position disposed along side the conduit housing;
e) bias means associated with the hinge to normally bias the end cap toward the opened position;
f) second objective optics mounted within the end cap;
g) a second CCD mounted within the end cap and positioned to receive an image focused on it by the second objective optics when the end cap is in the opened position with the first and second objective optics in a side-by-side relationship to provide stereoscopic images;
h) transmitter means connected to the first and second CCDs for transmitting image signals;
i) processing means for receiving the image signals and producing a visual stereoscopic representation of the image;
j) electrical power means mounted on the conduit housing for supplying electrical energy to the first and second CCDs and the transmitter means;
k) light means for emitting light onto an object to produce the image; and
l) locking means for locking the end cap in the closed position.

18. The stereoscopic electronic video endoscope of Claim 17 wherein the locking means comprises:
a) a channel extending longitudinally through the conduct housing;
b) a sleeve in the end cap aligned with the channel when the end cap is in the closed position; and
c) a locking rod extendable through the channel and having a distal end receivable in a frictional engagement with the sleeve to hold the end cap in the closed position against the force of the biasing means when its endoscope is inserted into a patient's body.

19. The stereoscopic electronic video endoscope of Claims 17 or 18 wherein the signal processing means includes a VCR.

20. The stereoscopic electronic video endoscope of any one of Claims 17 to 19 wherein the electrical power means is detachable from the conduit housing.

21. The stereoscopic electronic video endoscope of any one of Claims 17 to 20 wherein the CCD is positioned adjacent to the objective optics and is electrically connected to the transmitter means located in the proximal end of the conduit housing.

22. The endoscope of any one Claims 7 to 21 wherein the electrical power means comprises a battery.

23. The endoscope of Claim 22 wherein the battery is selected to withstand the temperature of steam autoclave sterilization.

24. The endoscope of any one of Claims 7 to 23 wherein objective optics, CCD means, transmitter means, electrical power means and light means are selected to withstand the temperature of steam autoclave sterilization.

25. The endoscope of any one of Claims 7 to 24 wherein the light means comprises fiber optic means selected to withstand the temperature of steam autoclave sterilization.
